(19) Europäisches Patentamt
European Patent Office
Office européen des brevets

(11) Veröffentlichungsnummer: **0 189 577**
**B1**

(12) # EUROPÄISCHE PATENTSCHRIFT

(45) Veröffentlichungstag der Patentschrift: **13.12.89**

(51) Int. Cl.⁴: **C07D 305/12**, C07D 309/30

(21) Anmeldenummer: **85116098.6**

(22) Anmeldetag: **17.12.85**

(54) **Verfahren zur Herstellung von Oxetanonen.**

(30) Priorität: **21.12.84 CH 6101/84**
**19.09.85 CH 4067/85**

(43) Veröffentlichungstag der Anmeldung:
**06.08.86 Patentblatt 86/32**

(45) Bekanntmachung des Hinweises auf die Patenterteilung:
**13.12.89 Patentblatt 89/50**

(84) Benannte Vertragsstaaten:
**AT BE CH DE FR GB IT LI LU NL SE**

(56) Entgegenhaltungen:
**EP-A- 0 129 748**
**FR-A- 2 426 679**

**THE JOURNAL OF ANTIBIOTICS, Band XXXI, Nr. 8, August 1978, Seiten 797-800; S. KONDO et al.: "The structure of esterastin, an inhibitor of esterase"**

(73) Patentinhaber: **F. HOFFMANN-LA ROCHE AG, Postfach 3255, CH-4002 Basel(CH)**

(72) Erfinder: **Barbier, Pierre, Dr., Rue de Lattre de Tassigny 41, F-68170 Rixheim(FR)**
Erfinder: **Schneider, Fernand, Dr., Marignanostrasse 28, CH-4059 Basel(CH)**
Erfinder: **Widmer, Ulrich, Dr., Alleeweg 13, CH-4310 Rheinfelden(CH)**

(74) Vertreter: **Lederer, Franz, Dr. et al, Van der Werth, Lederer & Riederer Patentanwälte Lucile-Grahn-Strasse 22, D-8000 München 80(DE)**

**EP 0 189 577 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von Oxetanonen der Formel

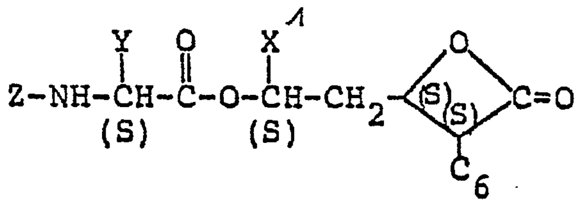

I

worin $X^1$ Undecyl oder 2Z,5Z-Undecadienyl, $C_6$ n-Hexyl, Y Isobutyl und Z Formyl, oder Y Carbamoylmethyl und Z Acetyl ist.

Diejenigen Verbindungen der Formel I, worin Y Isobutyl und Z Formyl ist, sind neu. Diese neuen Verbindungen zeichnen sich durch wertvolle pharmakologische Eigenschaften aus. Sie hemmen insbesondere die Pankreaslipase und können demgemäß zur Bekämpfung oder Verhütung von Krankheiten, insbesondere von Obesitas, Hyperlipämie, Atherosklerose und Arteriosklerose, Verwendung finden.

Das erfindungsgemäße Verfahren ist dadurch gekennzeichnet, daß man

a) eine Säure der Formel

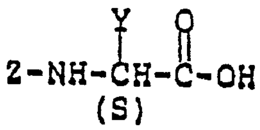

II

mit einem Alkohol der Formel

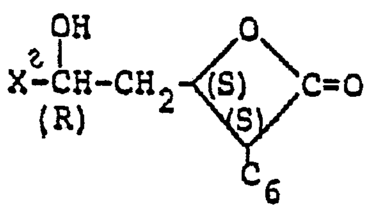

III

worin $X^2$ 3-Undecenyl oder wie $X^1$ ist, und X, $C_6$, Y und Z die obige Bedeutung haben, verestert, und ein gegebenenfalls erhaltenes Oxetanon der Formel

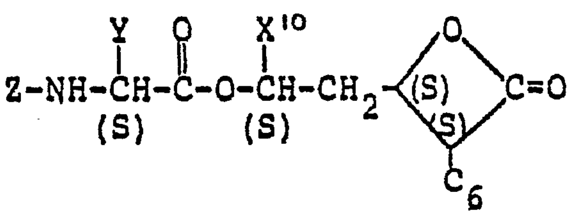

I'

worin $X^{10}$ 3-Undecenyl ist und $C_6$, Y und Z die obige Bedeutung haben, hydriert, oder

b) ein Oxetanon der Formel

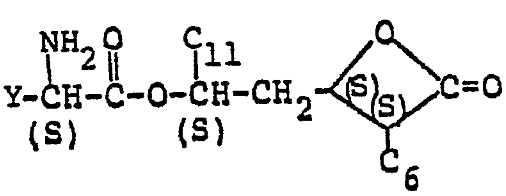

I''

worin $C_{11}$ Undecyl ist und Y und $C_6$ die obige Bedeutung haben, mit einem die Gruppe Z einführenden Alkanoylierungsmittel behandelt.

In J. of Antibiotics XXXI 1978, 797 ist zwar beschrieben, wie man im Esterastin – der Verbindung der obigen Formel I, worin $X^1$ 2Z,5Z-Undecadienyl, Y Carbamoylmethyl und Z Acetyl ist – die Gruppe $X^1$ zur Undecylgruppe unter Bildung des Tetrahydroesterastins (THE) hydriert, nicht aber wie man THE nach der obigen Verfahrensvariante a), gegebenenfalls über ein Oxetanon der Formel I' herstellen kann.

Die Veresterung a) kann in einem Lösungsmittel, z.B. einem Äther, wie Tetrahydrofuran (THF), in Gegenwart von Triphenylphosphin und Azodicarbonsäurediäthylester durchgeführt werden. Die Temperatur ist nicht kritisch; vorzugsweise arbeitet man bei Raumtemperatur.

Die Hydrierung eines als Zwischenprodukt erhaltenen Oxetanons I' kann in einem Lösungsmittel, z.B. einem Äther, wie THF, in Gegenwart eines Hydrierungskatalysators, wie Palladium auf Kohle, vorzugsweise bei etwa Raumtemperatur durchgeführt werden.

Die Alkanoylierung b) kann man in Gegenwart eines Säureanhydrids, z.B. eines gemischten Säureanhydrids, wie Ameisensäureessigsäureanhydrid, in einem Lösungsmittel, z.B. einem Äther, wie THF, vorzugsweise bei Raumtemperatur, bewerkstelligen.

Die Alkohole III kann man durch Abspaltung der Äthergruppe L in einem Äther der Formel

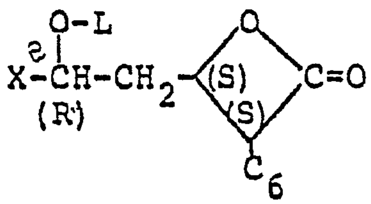

IV

herstellen, worin $C_6$ und $X^2$ die obige Bedeutung hat und L eine leicht spaltbare Äthergruppe, wie Tetrahydro-2H-pyran-2-yl, 1-Äthoxyäthyl oder t-Butyldimethylsilyl ist.

Diese Abspaltung kann man in einem Lösungsmittel, z.B. einem Alkohol, wie Äthanol, in Gegenwart eines sauren Katalysators, wie Pyridinium-4-toluolsulfonat, unter Erhitzen, z.B. auf 50–65°C durchführen.

Die Äther IV kann man durch Cyclisierung der Verbindung der Formel

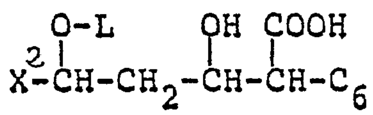

V

herstellen. Diese Reaktion kann man in einem Lösungsmittel, wie Pyridin, unter Abkühlen, z.B. auf 0°C, in Gegenwart von Benzolsulfochlorid durchführen.

Die Säuren V kann man herstellen entweder durch Verseifung entsprechender Ester der Formel

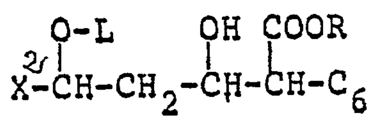

VI

worin R geradkettiges oder verzweigtes $C_{1-4}$-Alkyl, wie Methyl, Äthyl oder t-Butyl, ist und L und $X^2$ die obige Bedeutung haben,

oder, falls $X^2$ ein 2Z,5Z-Undecadienylrest ist, auch durch Kondensation von Octansäure mit einem Aldehyd der Formel

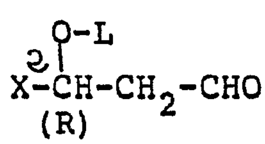

VII

Die Verseifung der Ester VI kann man mit einer alkoholischen Alkalimetallhydroxydlösung, wie einer methanolischen Kaliumhydroxydlösung, durch Erhitzen bei einer Temperatur bis zur Rückflußtemperatur des Reaktionsgemisches durchführen.

Die Kondensation des Aldehyds VII mit Octansäure kann man in einem Lösungsmittel, wie THF, in Gegenwart von Diisopropylamin und Butyllithium, unter Abkühlen, z.B. auf -50°C durchführen.

Die Säuren der Formel V, die in (5R)- oder (5S)-Form vorliegen, können in folgender Weise in die (2S, 3S,5R)- bzw. (2R,3R,5S)-Stereoisomeren übergeführt werden:

Man cyclisiert eine 5R- oder 5S-Säure der Formel V, z.B. unter Erhitzen auf 50–60°C in Äthanol mittels Toluol-4-sulfonsäuremonohydrat, zum entsprechenden 6R- bzw. 6S-Pyranolon der Formel

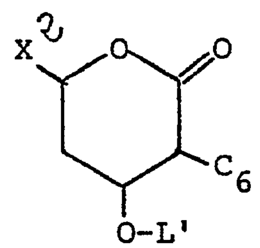

V-A

worin L' Wasserstoff ist und $X^2$ und $C_6$ die obige Bedeutung haben.

Dieses (6R)- oder (6S)-Pyranolon wird dann, z.B. in Aceton mittels Jones-Reagenz bei einer Temperatur unterhalb von 25°C, zum entsprechenden Pyrandion oxydiert und letzteres, z.B. in Essigester in Gegenwart von Platindioxyd, stereospezifisch zum (3S,4S,6R)- bzw. (3R,4R,6S)-Pyranolon der Formel V–A, worin L' Wasserstoff ist, hydriert. Dieses Pyranolon wird in eine Verbindung der Formel V–A, worin L' eine Ätherschutzgruppe, wie t-Butyldimethylsilyl, z.B. in Dimethylformamid mittels t-Butyldimethylchlorsilan, übergeführt. Der erhaltene cyclische (3S,4S,6R)- oder (3R,4R,6S)-Äther wird, z.B. durch Umsetzung mit einer wäßrigen Kaliumhydroxydlösung in Dioxan, aufgespalten und die entstandene Verbindung in situ in einen (2S,3S,5R)- bzw. (2R,3R,5S)-Äther der Formel

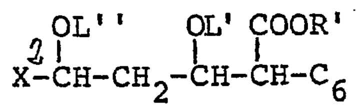

V-B

übergeführt, worin L" Wasserstoff, L' die gleiche Ätherschutzgruppe wie in Äther V–A, R' Benzyl oder p-Nitrobenzyl ist, und $X^2$ und $C_6$ die obige Bedeutung haben. Der erhaltene Äther V–B wird dann in einen Diäther der gleichen Formel übergeführt, worin L" eine Ätherschutzgruppe, wie Tetrahydro-2H-pyran-2-yl ist. Nach Abspaltung zunächst der Ätherschutzgruppe L', z.B. mit Tetrahydrobutylammoniumfluorid-trihydrat in THF, und dann der Gruppe R', z.B. durch Hydrierung in THF in Gegenwart von Pd/C, erhält man die erwünschte (2S,3S,5R)- bzw. (2R,3R,5S)-Säure der Formel V.

Die Ester VI kann man herstellen entweder durch n-Hexylierung der entsprechenden Ester der Formel

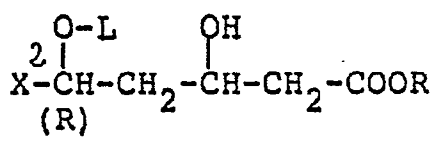

VIII

oder durch Reduktion der β-Ketoester der Formel

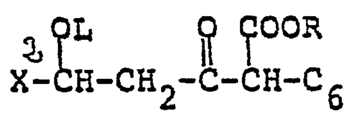

IX

Die n-Hexylierung der Ester VIII kann man durch Reaktion des Esters VIII in einem Lösungsmittel, wie THF, mit einer Lösung von n-Butyllithium in n-Hexan in Gegenwart von Diisopropylamin bei etwa –50°C, und anschließende Reaktion mit einer Lösung eines Hexylhalogenids wie 1-Bromhexan, in Hexamethylphosphorsäuretriamid bei einer Temperatur von etwa 0 bis 10°C durchführen.

Die Reduktion der β-Ketoester IX kann man gegebenenfalls in einem inerten Gas, wie Argon, in einem Lösungsmittel wie einem Äther, z.B. THF, mit einem komplexen Metallhydrid wie Natriumborohydrid ($NaBH_4$) bei einer Temperatur unterhalb von etwa 0°C durchführen.

Die Ester VIII kann man durch reduktive Spaltung eines Sulfoxyds der Formel

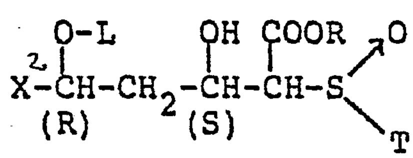

X

worin T p-Tolyl ist und L, R und $X^2$ die obige Bedeutung haben,

herstellen. Diese Reduktion kann man in einem Lösungsmittel, wie THF und Wasser, mittels Aluminiumamalgam durchführen.

Die β-Ketoester IX kann man durch Umsetzung eines Aldehyds der Formel $X^2$–CHO mit einem 2-Acetyloctansäure-$C_{1-4}$-alkylester und Verätherung des erhaltenen Alkohols der Formel

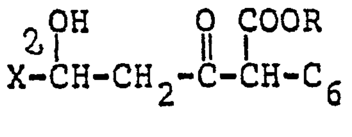

XI

erhalten.

Die Überführung des Aldehyds $X^2$–CHO in den Alkohol XI kann man wie im Beispiel 1L. beschrieben durchführen.

Die Sulfoxyde X kann man herstellen durch Kondensation eines Aldehyds der Formel VII mit einem Ester der Formel

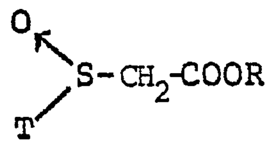

XII

z.B. wie in Beispiel 1I. beschrieben.

Die Aldehyde der Formel VII kann man durch Reduktion der entsprechenden Ester der Formel

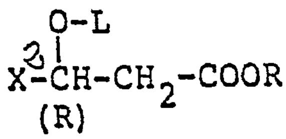

XIII

herstellen, z.B. mit Diisobutylaluminiumydrid in einem Lösungsmittel wie Toluol, bei einer Temperatur von etwa -60 bis -80°C.

Die Ester der Formel XIII kann man herstellen ausgehend von den Aldehyden der Formel $X^2$–CHO über die Sulfoxyde der Formel

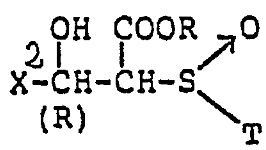

XIV

und die Alkohole der Formel

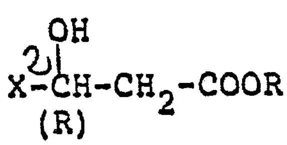

XV

z.B. wie beschrieben in den Beispielen 1H.b), 1I.b) und 1K.a).

Die Ausgangsoxetanone der Formel I' werden in analoger Weise wie die Oxetanone der Formel I ausgehend von Estern der Formel XIII, worin $X^2$ ein 3-Undecenylrest ist, über die entsprechenden Verbindungen der Formeln III–XI hergestellt.

Die Ausgangsoxetanone der Formel I" kann man herstellen durch Abspaltung der Aminoschutzgruppe W in einem Oxetanon der Formel

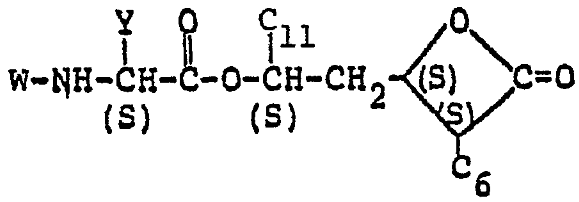

I'''

worin $C_{11}$, Y und $C_6$ die obige Bedeutung hat.

Als Beispiel einer Aminoschutzgruppe W kann man Benzyloxycarbonyl und p-Nitrobenzyloxycarbonyl nennen. Die Abspaltung von W kann man durch Hydrierung in einem Lösungsmittel, z.B. einem Äther, wie THF, in Gegenwart eines Hydrierungskatalysators, wie Palladium auf Kohle, vorzugsweise bei Raumtemperatur durchführen. Eine in I''' vorhandene Undecadienylgruppe X wird bei der hydrogenolytischen Abspaltung von W zur Undecylgruppe hydriert.

Die Oxetanone der Formel I''' kann man herstellen durch Veresterung eines Alkohols der Formel

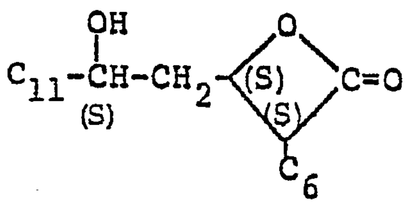

III'

mit einem durch Umsetzung einer Säure der Formel

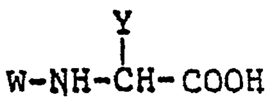

II'

mit Dicyclohexylcarbodiimid oder N-Äthyl-N'-(3-dimethylaminopropyl)-carbodiimid-hydrochlorid erhaltenen Säureanhydrid. Die Herstellung dieses Säureanhydrids kann unter Abkühlung, z.B. auf 2–3°C, in einem Lösungsmittel, wie Methylenchlorid, durchgeführt werden und die darauf folgende Veresterung in einem Lösungsmittel, wie Dimethylformamid.

Die Alkohole der Formel III' sind die (S)-Epimeren der Alkohole der Formel III, worin $X^2$ Undecyl ist, und können in ähnlicher Weise ausgehend von den Aldehyden der Formel $C_{11}$–CHO über die (S)-Enantiomeren der Ester der Formel XV, über die (S)-Enantiomeren der Äther der Formel XIII und VII, und über die (S)-Epimeren der Verbindungen der Formeln VIII, VI, V und IV hergestellt werden.

Zur Überführung der Aldehyde der Formel $X^2$–CHO bzw. der (S)-Enantiomeren der Aldehyde der Formel VII in die entsprechenden (S)-Enantiomeren der Ester der Formeln XV bzw. VIII kann man an Stelle eines Sulfinylesters XII das (R)-α-(Hydroxydiphenylmethyl)benzylacetat verwenden. In diesem Fall erhält man intermediär an Stelle der Sulfoxyde der Formeln XIV bzw. X die dem Alkylester der Formeln XV bzw. VIII entsprechenden (R)-2-Hydroxy-1,2,2-triphenyläthylester.

Die Ester der Formel

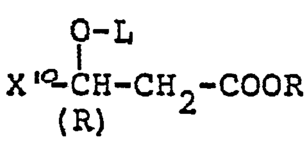

XIII-A

kann man wie die Ester XIII herstellen, oder ausgehend von einem Hexensäureester der Formel

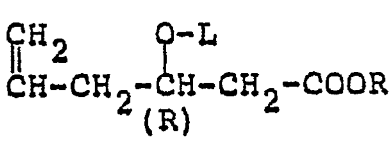

XVI

über ein Aldehyd der Formel

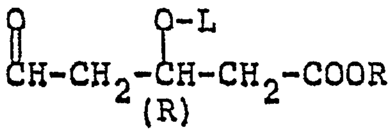

XVII

z.B. wie beschrieben im Beispiel 1M.

Beispiel 1

1A. Herstellung eines Oxetanons der Formel I:

1A.a) Eine Lösung von 100 mg rac-3-Hexyl-4-(2-hydroxy-tridecyl)-2-oxetanon(2R,3S,4S:2S,3R,4R) bzw. von 100 mg (3S,4S)-3-Hexyl-4-[(R)-2-hydroxytridecyl]-2-oxetanon, 74 mg Triphenylphosphin und 45 mg N-Formyl-L-leucin in 2 ml THF tropft man unter Rühren 44,3 µl Azodicarbonsäurediäthylester zu. Nach Rühren über Nacht wird die organische Phase im Vakuum eingedampft und der Rückstand durch Chromatographie an Kieselgel mit Toluol-Essigsäureäthylester (9:1) gereinigt. Man erhält 20 bzw. 37 mg N-Formyl-L-leucin(S)-1-[[(2S,3S)-3-hexyl-4-oxo-2-oxetanyl]methyl]dodecylester, $[\alpha]^{20}_D$ =-33° (c = 0,36, $CHCl_3$).

1A.b) Eine Lösung von 2,5 mg N-Formyl-L-leucin (S,4Z)-1-[[(2S,3S)-3-hexyl-4-oxo-2-oxetanyl]methyl] -4-dodecenylester in 0,1 ml THF wird mit 1 mg Palladium auf Kohle versetzt, dann hydriert man während 3 Stunden. Den Katalysator filtriert man ab und chromatographiert mit Toluol-Essigsäureäthylester (9:1) über Kieselgel. Man erhält 1,5 mg (N-Formyl-L-leucin (S)-1-[[(2S,3S)-3-hexyl-4-oxo-2-oxetanyl]methyl] -4-dodecylester, $^{+}$H-NMR-Spektrum (270 MHz, $CDCl_3$): 0,89 (6H); 0.97 (6H); 1,15–2,25 (34H); 3,22 (2H); 4,29 (1H); 4,70 (1H); 5,04 (1H); 5,91 (1H); 8,23 (1H) ppm.

1A.c) Analog a) erhält man

1) N-Acetyl-3-carbamoyl-L-alanin(S)-1-[[(2S,3S) -3-hexyl-4-oxo-2-oxetanyl]methyl]dodecylester, MS: $M^+$ (510); $(M+1)^+$ (511)

2) N-Formyl-L-leucin(S)-1-[[(2S,3S) -3-hexyl-4-oxo-2-oxetanyl]methyl]-3Z,6Z-dodecadienylester, $[\alpha]^{20}_D$=–19,4° (c 0,28, $CHCl_3$); MS: $(M+1)^+$ (492)

3) N-Formyl-L-leucin(S,4Z)-1-[[(2S,3S)-3-hexyl -4-oxo-2-oxetanyl]methyl]-4-dodecenylester.

1B. Herstellung der Ausgangsalkohole der Formel III

1B.a) 265 mg eines Isomerengemisches von 3-Hexyl-4-[2-[(tetrahydro-2H-pyran-2-yl)oxy]tridecyl] -2-oxetanon werden in 2,5 ml Aethanol gelöst und 13 mg Pyridinium-4-toluolsulfonat werden zugesetzt. Das Reaktionsgemisch wird auf 55-60°C erhitzt, bis die Reaktion beendet ist. Das Lösungsmittel wird im Vakuum entfernt und der Rückstand in Aether aufgenommen, wobei Kristalle ausfallen, die durch Filtration entfernt werden. Das Lösungsmittel wird im Vakuum abgedampft und der Rückstand an Kieselgel chromatographiert. Man erhält das rac-3-Hexyl-4-(2-hydroxytridecyl-2-oxetanon(2R,3S,-4S:2S,3R,4R), MS: $M^+$ (354), Smp. 44,5–46°C.

Auf analoge Weise erhält man:

1B.b) das (3S,4S)-3-Hexyl-4-[(R)-2-hydroxytridecyl]-2-oxetanon, Smp. 46–46,5°C
1B.c) das (3S,4S)-3-Hexyl-4-[(R)-2-hydroxy-4Z,7Z-tridecadienyl]-2-oxetanon
1B.d) das trans-3-Hexyl-4-[(R)-2-hydroxy-5Z-tridecenyl] -2-oxetanon.

1C. Herstellung der Aether der Formel IV

1C.a) 0,7 g eines Isomerengemisches von 2-Hexyl-3-hydroxy-5-[(tetrahydro-2H-pyran-2-yl)oxy]-hexadecansäure werden in 15 ml Pyridin gelöst und auf 0°C abgekühlt. Nach tropfenweiser Zugabe von 0,4 ml Benzolsulfochlorid wird bei 0°C gerührt. Die Reaktionsmischung wird auf 120 ml 10%-iger Kochsalzlösung gegossen und dreimal mit 30 ml Diäthyläther extrahiert. Die vereinigten Extrakte werden getrocknet, filtriert und eingedampft. Nach Chromatographie an Kieselgel erhält man ein Isomerengemisch von 3-Hexyl-4-[2-[(tetrahydro-2H-pyran -2-yl)oxy]tridecyl]-2-oxetanon, MS: $(M+1)^+$ (440).

Auf analoge Weise erhält man:

1C.b) 3-Hexyl-4[(R)-2-[(tetrahydro-2H-pyran-2-yl)oxy]tridecyl]-2-oxetanon
1C.c) 3-Hexyl-4[(R)-2-[(tetrahydro-2H-pyran-2-yl)oxy]4Z,7Z-tridecadienyl]-2-oxetanon
1C.d) 3-Hexyl-4-[(R)-2-[(tetrahydro-2H-pyran-2-yl)oxy]-5Z-tridecenyl-2-oxetanon.

1D. Herstellung der Säuren der Formel V

1D.a) 1 g eines Diastereomerengemisches von 2-Hexyl-3-hydroxy(R) -5-[(tetrahydro-2H-pyran-2-yl)-oxy]hexadecansäure-t-butylester wird in 17 ml einer 2N methanolischer Kaliumhydroxidlösung unter Rückfluss erhitzt. Die Reaktionsmischung wird abgekühlt und auf 60 ml Eiswasser gegossen. Durch tropfenweise Zugabe von 1N wässriger Salzsäure wird ein pH von 1 eingestellt, dann wird mit Aether extrahiert. Die vereinigten Aetherphasen werden getrocknet, filtriert und eingedampft. Das Oel wird an Kieselgel chromatographiert. Man erhält ein Diastereomerengemisch von 2-Hexyl-3-hydroxy(R)-5-[(tetrahydro -2H-pyran-2-yl)oxy]hexadecansäure.

Auf analoge Weise erhält man:

1D.b) 2-Hexyl-3-hydroxy-5-[(tetrahydro-2H-pyran-2-yl)oxy]hexadecansäure
1D.c) 2-Hexyl-3-hydroxy(R)-5-[(tetrahydro-2H-pyran-2-yl)oxy]-7Z,10Z-hexadecadensäure.

1E. Herstellung der Säuren der Formel V (Variante)

1E.a) 2 ml Diisopropylamin in 30 ml trockenem THF werden auf -20°C abgekühlt. Dann werden 9,68 ml Butyllithium (1,6M/Hexan) zugetropft, so dass die Temperatur -20°C nicht übersteigt. Anschliessend wird 15 Minuten gerührt und dann auf -50°C abgekühlt. Danach werden 1,11 ml Octansäure in 10 ml THF zugetropft und 10 Minuten weiter bei -50°C gerührt. Man rührt eine Stunde bei Raumtemperatur und kühlt anschliessend wieder auf -50°C. Nun werden 2 g (R)-3-[(Tetrahydro-2H-pyran-2-yl)oxy]5Z,8Z-tetradecadienal in 10 ml THF zugetropft. Man rührt 30 Minuten bei -50°C, dann 72 Stunden bei Raumtemperatur. Nach Hydrolyse mit 2N Salzsäure wird das Reaktionsgemisch eingedampft. Der Rückstand wird mit Aether extrahiert. Die organische Phase wird getrocknet, filtriert und eingedampft. Das erhaltene Material wird durch Kieselgel filtriert. Man erhält die 2-Hexyl-3-hydroxy(R)-5-[(tetrahydro-2H-pyran -2-yl)oxy]7Z,10Z-hexadecadiensäure.
1E.b) Auf analoge Weise erhält man:
die 2-Hexyl-3-hydroxy(R)-5-[(tetrahydro -2H-pyran-2-yl)oxy]-8Z-hexadecenylcarbonsäure.

1F. Herstellung der Ester der Formel VI

1F.a) 1,8 ml Diisopropylamin werden unter Argon auf -5°C abgekühlt, 8,7 ml einer 1,6N n-Butyllithiumlösung in n-Hexan werden zugetropft. Danach wird 10 Minuten gerührt. Nach Kühlung auf -50°C wird das Kühlbad entfernt. Man tropft eine Lösung von 2,67 g eines Diastereomerengemisches von 3-Hydroxy(R)-5-[(tetrahydro -2H-pyran-2-yl)oxy]hexadecansäure-t-butyleste in 9 ml THF zu. Die Temperatur steigt dabei auf -20°C. Man lässt auf 0°C aufwärmen und rührt 10 Minuten. Dann wird eine Lösung von 0,93 ml 1-Bromhexan in 4,4 ml Hexamethylphosphorsäuretriamid zugegeben. Die Temperatur steigt auf 9°C an. Danach lässt man auf Raumtemperatur aufwärmen und rührt 2 1/2 Stunden. Die Lösung wird auf 200 ml Eiswasser gegossen und mit Kochsalz gesättigt. Man extrahiert mit Aether. Die vereinigten Extrakte werden getrocknet, filtriert und eingedampft. Das zurückbleibende Oel wird an Kieselgel chromatographiert. Man erhält den 2-Hexyl-3-hydroxy(R)-5-[(tetrahydro -2H-pyran-2-yl)oxy]hexadecansäure-t-butylester, MS: $(M-O-t-Butyl)^+$ (439).
1F.b) Auf analoge Weise erhält man:
den 2-Hexyl-3-hydroxy(R)-5-[(tetrahydro-2H-pyran-2-yl)oxy]-7Z,10Z-hexadecadiensäure-t-butylester.

1G. Herstellung der Ester der Formel VI (Variante)

Unter Argonbegasung werden 7,76 g 2-Hexyl-3-oxo-5-[(tetrahydro-2H-pyran -2-yl)oxy]hexadecansäure-methylester in 500 ml THF gelöst, mit 20 ml Aethanol versetzt und auf -5°C abgekühlt. Unter Rühren werden 5,3 g $NaBH_4$ portionenweise zugegeben, so dass die Temperatur 0°C nicht übersteigt. Nach 3 Stunden Rühren wird das überschüssige Natriumborhydrid abfiltriert, das Reaktionsgemisch wird mit 2N Salzsäure in der Kälte hydrolysiert (bis pH 6) und das Lösungsmittel wird abgedampft. Der Rückstand wird mit Aether extrahiert und die ätherische Phase wird getrocknet und abgedampft. Man erhält 7,71 g 2-Hexyl-3-hydroxy-5-[(tetrahydro-2H-pyran -2-yl)oxy]hexadecansäure-methylester.

1H. Herstellung der Ester der Formel VIII und XV

13,6 g eines Diastereomerengemisches von 3-Hydroxy(R)-5-tetrahydro-2H-pyran -2-yl)oxy]-2-[(S)-p-tolylsulfinyl]hexadecansäure -t-butylester werden in ein Gemisch von 6 l THF und 0,6 l Wasser gelöst. Dann werden innerhalb von 6 Stunden 150 g amalgamierte Aluminiumfolie portionenweise zugesetzt. Dabei wird die Temperatur zwischen 15°C und 20°C gehalten. Nach beendeter Zugabe wird solange gerührt, bis die Reaktion beendet ist. Das unlösliche Material wird abgesaugt und zuerst mit 1 l, dann mit 2 l THF gewaschen. Der Filterkuchen wird in 2 l Diäthyläther aufgenommen, gut verrührt und erneut abgesaugt. Dieses Prozedere wird einmal wiederholt. Die vereinigten organischen Phasen werden eingedampft und der ölige Rückstand wird durch Chromatographie an Kieselgel gereinigt. Man erhält den 3-Hydroxy(R)-5-[(tetrahydro-2H-pyran -2-yl)oxy]hexadecansäure-t-butylester, MS: (M-O-t-Butyl)+ (355).

Auf analoge Weise erhält man:

1H.a) den 3-Hydroxy(R)-5-[(tetrahydro -2H-pyran-2-yl)oxy]7Z,-10Z-hexadecadiensäure-t-butylester

1H.b) den (R)-3-Hydroxy-5Z,8Z-tetradecadiensäure-t-butylester, $[\alpha]^{20}_D$= -12,67° ( c = 15, $CHCl_3$).

1I. Herstellung der Sulfoxyde der Formel X und XIV

16,5 g [(S)-p-Tolylsulfinyl]essigsäure-t-butylester werden in ein Gemisch von 600 ml Aether und 60 ml THF gelöst und auf -78°C abgekühlt. Dann werden 43 ml t-Butylmagnesiumbromid (2N Aetherlösung) innerhalb von 30 Minuten zugetropft, sodass die Temperatur nicht über -70°C steigt. Nach einer Stunde Rühren bei -78°C werden 13,4 g (R)-3-[(Tetrahydro-2H-pyran -2-yl)oxy]tetradecanal in 100 ml THF zugetropft und es wird noch 2 Stunden bei -78°C gerührt. Das Reaktionsgemisch wird mit 2N Salzsäure hydrolysiert und das Lösungsmittel wird abgedampft. Die verbleibende Reaktionsmischung wird mit Aether extrahiert und die ätherische Phase wird getrocknet und eingedampft. Das Rohprodukt wird durch eine Säule von Kieselgel eluiert. Man erhält 14,9 g 3-Hydroxy(R)-5-[(Tetrahydro-2H-pyran -2-yl)oxy]-2-[(S)-p-tolylsulfinyl]-hexadecansäure -t-butylester, Smp. 97-98°C.

Auf analoge Weise erhält man

1I.a) 3-Hydroxy(R)-5-[(tetrahydro-2H-pyran -2-yl)oxy]-2-[(S)-p-tolylsulfinyl]7Z,10Z-hexadecadiensäure-t-butylester

1I.b) (R)-3-Hydroxy-2-[(R)-p-tolylsulfinyl]5Z,7Z-tetradecadiensäure-t-butylester.

1J. Herstellung der Aldehyde der Formel VII

1J.a) Unter Argonbegasung und Feuchtigkeitsausschluss werden 9,2 g (R)-3-[(Tetrahydro-2H-pyran -2-yl)oxy]tetradecansäure-t-butylester in 115 ml Toluol gelöst und auf -75°C abgekühlt. Es werden dann innert 1/2 Stunde 26,5 ml einer 1,2M DIBAH Lösung in Toluol zugetropft, sodass die Temperatur -70°C nicht übersteigt. Dann rührt man noch eine Stunde bei -75°C. Es werden dann 7,4 ml gesättigte Ammoniumchloridlösung und anschliessend 15,5 ml 1N Salzsäure bei -70°C zugetropft. Das Kühlbad wird entfernt und man lässt auf Raumtemperatur aufwärmen. Nach einer Stunde Rühren bei Raumtemperatur wird die organische Phase abgetrennt und mit Wasser gewaschen. Die organische Phase wird getrocknet, filtriert und eingedampft. Das erhaltene Material wird an Kieselgel chromatographiert. Man erhält das [(R)-3-[(Tetrahydro-2H-pyran -2-yl)oxy]tetradecanal als farbloses Oel.

Auf analoge Weise erhält man:

1J.b) (R)-3-[(Tetrahydro-2H-pyran -2-yl)oxy]5Z,8Z-tetradecadienal

1J.c) (R)-3-[(Tetrahydro-2H-pyran -2-yl)oxy]-6Z-tetradecenal.

1K. Herstellung der Ester der Formel XIII und IX

1K.a) 3,02 g (R)-3-Hydroxy-5Z,8Z-tetradecadiensäure-t-butylester und 2,5 ml frisch destilliertes 3,4-Dihydro-2H-pyran werden in 300 ml Methylenchlorid gelöst und auf 3°C abgekühlt. Danach werden 40 mg p-Toluolsulfonsäuremonohydrat zugegeben, wobei die Temperatur auf 8°C ansteigt. Es wird gerührt, bis die Reaktion beendet ist. Darauf wird die Lösung mit einem Gemisch von 125 ml wässrig gesättigter Kochsalzlösung, wässrig 125 ml gesättigter Natriumhydrogencarbonat und 250 ml Wasser gewaschen. Nach Trocknung wird filtriert und das Lösungsmittel entfernt. Man erhält (R)-3-[(Tetrahydro-2H-pyran -2-yl)oxy]5Z,8Z-tetradecadiensäure-t-butylester.

1K.b) Auf analoge Weise erhält man:
den 2-Hexyl-3-oxo-5-[(Tetrahydro-2H-pyran -2-yl)oxy]hexadecansäuremethylester, Smp. 37°-38°C.

1L. Herstellung eines Alkohols der Formel XI

5 g einer 55%-igen Natriumhydriddispersion werden mit Hexan gewaschen und mit 600 ml THF versetzt. Unter Kühlen wird eine Lösung von 18,9 g 2-Acetyloctansäuremethylester in 80 ml THF zugetropft. Nach 2 Stunden Rühren kühlt man auf -10°C ab und versetzt unter Kühlung mit 65 ml Butyllithium (1,6 M/Hexan). Anschliessend lässt man die Reaktionsmischung 1 Stunde bei dieser Temperatur. Bei -10°C wird eine Lösung von 19,7 g Dodecanal in 80 ml THF zugetropft. Man lässt auf Raumtemperatur erwärmen und rührt 2 Stunden weiter. Das Reaktionsgemisch wird mit 100 ml 2N Salzsäure hydrolysiert und dann abgedampft. Der Rückstand wird mit Aether extrahiert und die ätherische Phase wird getrocknet und abgedampft. Nach Chromatographie auf Kieselgel erhält man 10,3 g 2-Hexyl-5-hydroxy-3-oxo-hexadecansäuremethylester, Smp. 38°-39°C.

1M. Herstellung eines Esters der Formel XIII-A

Eine Lösung von 0,51 g Diisopropylamin in 20 ml THF wird mit 3,13 ml einer 1,6 molaren Lösung von Butyllithium in Hexan bei 0°C versetzt. Dan kühlt man auf -78°C ab und gibt 2,3 g Heptyltriphenylphosphoniumbromid hinzu und lässt 5 Minuten bei dieser Temperatur. Man tropft anschliessend eine Lösung von 5-Formyl-(R)-3-[(tetrahydro-2H-pyran -2-yl)-oxy]pentancarbonsäureäthylester in 10 ml THF hinzu. Man lässt bei Zimmertemperatur über Nacht rühren. Das Reaktionsgemisch versetzt man mit Wasser, extrahiert mit Aether, trocknet und dampft im Vakuum ein. Den Rückstand chromatographiert man mit Toluol-Essigsäureäthylester (9:1) über Kieselgel und erhält 0,5 g (R)-3-[(Tetrahydro-2H-pyran -2-yl)oxy]-6Z-tetradecencarbonsäureäthylester.

1N. Herstellung eines Aldehydes der Formel XVII

Eine Lösung von 2,56 g (R)-3-[(Tetrahydro-2H-pyran -2-yl)oxy]-6-heptensäuremethylester in 40 ml Essigsäureäthylester wird bei -75°C mit Ozon behandelt. Nach beendeter Reaktion gibt man 0,1 g Pd auf Kohle hinzu und hydriert bei Zimmertemperatur. Nach beendeter Wasserstoffaufnahme filtriert man den Katalysator ab, wäscht mit Essigsäureäthylester und dampft im Vakuum ein. Man erhält rohen 5-Formyl-(R)-3-[(tetrahydro -2H-pyran-2-yl)oxy]-pentancarbonsäuremethylester.

1.O. Auftrennung der Säuren der Formel V in ihre Stereoisomere

1.O.a) 15,4 g eines Diastereomerengemisches von 2-Hexyl-3-hydroxy R-5-[(tetrahydro-2H-pyran -2-yl)oxy]hexadecansäure werden in 160 ml Aethanol gelöst und 800 mg Toluol-4-sulfonsäuremonohydrat werden zugesetzt. Das Reaktionsgemisch wird auf 55-60°C erhitzt bis die Reaktion beendet ist. Das Lösungsmittel wird im Vakuum entfernt und der Rückstand in 160 ml Dichlormethan gelöst. Man rührt eine Stunde bei Raumtemperatur. Das Reaktionsgemisch wird eingedampft. Das erhaltene Material wird an Kieselgel chromatographiert. Man erhält den Tetrahydro-3-hexyl-4-hydroxy R-6-undecyl-2H-pyran-2-on, Smp. 95-96°C.

1.O.b) 3 g eines Diastereomerengemisches von Tetrahydro-3-hexyl-4-hydroxy-(R)-6-undecyl-2H-pyran-2-on werden in 300 ml Aceton gelöst. Unter Rühren werden 3 ml Jones Reagens zugetropft, so dass die Temperatur 25°C nicht übersteigt. Nach 3 Stunden wird das Reaktionsgemisch auf 700 ml $H_2O$ gegossen. Das Lacton fällt aus und wird abfiltriert. Nach Umkristallisieren aus Aether/n-Hexan erhält man 1,7 g Tetrahydro-3-hexyl-4-oxo-(R)-6-undecyl-2H-pyran -2-on, Smp. 112,5-113,5°C.

1.O.c) 8 g eines Isomerengemisches von Tetrahydro-3-hexyl-4-oxo-(R)-6-undecyl-2H-pyran-2-on werden in 2 l Essigester gelöst und 3 g $PtO_2$ werden zugesetzt. Dann hydriert man (50 bar) während 12 Stunden. Der Katalysator wird abfiltriert und die Lösung eingedampft. Nach Umkristallisation erhält man 7 g (3S,4S,6R)-Tetrahydro-3-hexyl-4-hydroxy-6-undecyl-2H-pyran-2-on, Smp. 108-109°C.

1.O.d) 1,5 g (3S,4S,6R)-Tetrahydro-3-hexyl-4-hydroxy-6-undecyl-2H-pyran-2-on werden in 8 ml DMF gelöst. Dann wird 0,85 g t-Butyldimethylchlorsilan in 4 ml DMF zugetropft. Man rührt 48 Stunden. Das Reaktionsgemisch wird in 100 ml Aether gegossen und mit 1N Salzsäure gewaschen. Die organische Phase wird getrocknet, filtriert und eingedampft. Das erhaltene Material wird an Kieselgel chromatographiert. Man erhält 1,26 g (3S,4S,6R)-Tetrahydro-3-hexyl- -4-[(t-butyldimethylsilyl)oxy] -6-undecyl-2H-pyran-2-on, MS: 411 ($M^+$-t-Butyl).

1.O.e) 0,3 g (3S,4S,6R)-Tetrahydro-3-hexyl-4-[(t-butyl-dimethylsilyl)oxy] -6-undecyl-2H-pyran-2-on wird in ein Gemisch von 12 ml Dioxan und 0,64 ml 1N wässrigem Kaliumhydroxid gelöst. Man rührt über Nacht. Dann wird das Reaktionsgemisch eingedampft und in 10 ml Hexamethylphosphortriamid gelöst. Es werden 0,35 ml Benzylchlorid zugesetzt. Man rührt 2 Tage. Das Reaktionsgemisch wird auf Wasser gegossen und mit Aether extrahiert. Die Aetherphase wird getrocknet, filtriert und eingedampft. Das Oel wird an Kieselgel chromatographiert. Man erhält 330 mg (2S,3S,5R)-2-Hexyl-3-[t-butyldimethylsilyl)oxy] -5-hydroxyhexadecansäurebenzylester, MS: 519 ($M^+$-t-Butyl).

1.O.f) 350 mg (2S,3S,5R)-2-Hexyl-3-[(t-butyl-dimethylsilyloxy] -5-hydroxydecansäurebenzylester und 0,5 ml frisch destilliertes 3,4-Dihydro-2H-pyran werden in 10 ml Methylenchlorid gelöst und auf -15°C abgekühlt. Man gibt ein Kristal p-Toluolsulfonsäuremonohydrat zu. Es wird gerührt bis die Reaktion beendet ist. Darauf wird die Lösung eingedampft und der Rückstand auf Kieselgel chromatographiert. Man erhält 330 mg (2S,3S,5R)-2-Hexyl-3[(tert-butyl-dimethylsilyl)oxy] -5-[(tetrahydro-2H-pyran-2-yl)oxy]hexadecansäurebenzylester, MS: 603 ($M^+$-t-Butyl).

1.O.g) 480 mg (2S,3S,5R)-2-Hexyl-3[(t-butyl-dimethylsilyl)oxy] -5-[(tetrahydro-2H-pyran-2-yl)oxy]-hexadecansäurebenzylester und 350 mg Tetrabutylammoniumfluorid-Trihydrat werden in 8 ml THF gelöst und 12 Stunden gerührt. Nach Eindampfen wird der Rückstand in 50 ml Aether gelöst und mit Wasser gewaschen. Die ätherische Phase wird getrocknet und eingedampft. Das Rohprodukt wird an Kieselgel chromatographiert. Man erhält 240 mg (2S,3S,5R)-2-Hexyl-3-hydroxy-5-[(tetrahydro-2H-pyran-2-yl)oxy]hexadecansäurebenzylester, MS: 463 [$(M+H)^+$-Dihydro-2H-pyran-2-yl).

1.O.h) 430 mg (2S,3S,6R)-2-Hexyl-3-hydroxy-5-[(tetrahydro-2H-pyran-2-yl)oxy]hexadecansäurebenzylester in 10 ml THF werden mit Pd/C 10% versetzt und 3 Stunden hydriert. Den Katalysator filtriert man ab und nach Eindampfen wird das Rohprodukt an Kieselgel chromatographiert. Man erhält

(2S,3S,5R)-2-Hexyl-3-hydroxy-5-[(tetrahydro -2H-pyran-2-yl)oxy]hexadecansäure.

Beispiel 2

2A. Herstellung eines Oxetanons der Formel I

Man löst 9 mg (S)-Leucin-1-[(2S,3S)-3-hexyl -4-oxo-2-oxetanyl)methyl]dodecylester in 0,3 ml THF und gibt 1,6 µl Ameisensäureessigsäureanhydrid zu. Die Reaktion ist in kurzer Zeit beendet. Man gibt 3 ml Diäthyläther zu und wäscht mit 2%-iger Natriumhydrogencarbonatlösung. Dann trocknet man über Natriumsulfat, filtriert und dampft ein. Der Rückstand wird an Kieselgel chromatographiert. Man erhält N-Formyl-(S)-leucin-(S)-1-[[(2S,3S) -3-hexyl-4-oxo-2-oxetanyl]methyl]dodecylester, $[\alpha]^{25}_D$ = -31,9° (c = 0,345, $CHCl_3$).

2B. Herstellung eines Oxetanons der Formel I''

12 mg N-[(Benzyloxy)carbonyl]-L-leucin (S)-1-[[(2S,3S)-3-hexyl-4-oxo-2-oxetanyl]methyl]dodecylester werden in 0,5 ml THF gelöst und in Gegenwart von 5 mg 10% Pd/C bei Raumtemperatur hydriert. Nach beendeter Reaktion wird der Katalysator abfiltriert und eingedampft. Das Produkt, (S)-Leucin-1-[[(2S,3S)-3-hexyl -4-oxo-2-oxetanyl)methyl]dodecylester, wird direkt in die Formylierungsreaktion 2A. eingesetzt.

2C. Herstellung eines Oxetanons der Formel I'''

45 mg N-[(Benzyloxy)carbonyl]-L-leucin werden unter Feuchtigkeitsausschluss in 0,5 ml Methylenchlorid gelöst und auf 2-3°C abgekühlt. Man setzt 17 mg Dicyclohexylcarbodiimid zu und rührt 15 Minuten. Die weissen Kristalle werden abfiltriert und das Filtrat wird eingedampft. Der Rückstand wird in 0,5 ml N,N-Dimethylformamid gelöst und diese Lösung zu 27 mg (3S,4S)-3-Hexyl-4-[(S)-2-hydroxy-tridecyl] -2-oxetanon und 1 mg 4-Dimethylaminopyridin in 0,5 ml DMF hinzugegeben. Dann wird mit Wasser verdünnt und mit Diäthyläther extrahiert. Die vereinigten organischen Phasen werden über Natriumsulfat getrocknet, filtriert und eingedampft. Nach Chromatographie an Kieselgel erhält man den N-[(Benzyloxy)carbonyl]-L-leucin (S)-1-[[(2S,3S)-3-hexyl-4-oxo-2-oxetanyl]methyl]dodecylester als weisse Kristalle vom Schmelzpunkt 43-46°C.

2D. Herstellung eines Alkohols der Formel III'

1,23 g (3S,4S)-4-[(S)-2-(tert-Butyldimethylsiloxy)tridecyl] -3-hexyl-2-oxetanon werden in 6 ml Methanol gelöst und unter Zusatz von 1,05 g DOWEX 50W-X8 (saurer Kationenaustauscher auf der Basis von Sulfonsäurereste enthaltendem polymerem Divinylbenzol) zum Rückfluss erhitzt. Der Ionenaustauscher wird abfiltriert und das Filtrat eingedampft. Der Rückstand wird an Kieselgel chromatographiert. Man erhält nach Umkristallisation aus Hexan das [3S,4S]-3-Hexyl-4-[(S)-2-hydroxytridecyl] -2-oxetanon vom Schmelzpunkt 63-64°C.

2E. Herstellung eines Aethers der Formel IV (in (S)-Form)

In Analogie zu Beispiel 1C.a) erhält man (3S,4S)-4-[(S)-2-(tert.Butyldimethylsiloxy)tridecyl] -3-hexyl-2-oxetanon, welches etwa zu 20% ein (cis)-4-[2-tert-Butyldimethylsiloxy)tridecyl] -3-hexyl-2-oxetanon enthält, aus einem Diastereomerengemisch, welches hauptsächlich (2S,3S,5S)-5-(tert-Butyldimethylsiloxy) 2-hexyl-3-hydroxy-hexadecansäure enthält.

2F. Herstellung der Säure der Formel V (in (S)-Form)

430 mg eines Diastereomerengemisches, welches vornehmlich aus (2S,3S,5S)-5-(t-Butyldimethylsiloxy) -2-hexyl-3-hydroxyhexadecansäuremethylester besteht, werden in 8,6 ml 2N methanolischer Kaliumhydroxidlösung aufgenommen und bis zur Beendigung der Reaktion gerührt. Das Reaktionsgemisch wird auf Wasser gegossen und durch Zugabe von 2N Salzsäure angesäuert. Nach mehrmaliger Extraktion mit Diäthyläther werden die vereinigten Extrakte getrocknet, filtriert und eingedampft. Der Rückstand wird an Kieselgel chromatographiert. Die so erhaltene Carbonsäure wird direkt weiterverarbeitet.

2G. Herstellung eines Esters der Formel VI (in (S)-Form)

In Analogie zu Beispiel 1F.a) erhält man ein Diastereomerengemisch, welches hauptsächlich aus (2S,3S,5S)-5-(t-Butyldimethylsiloxy) -2-hexyl-3-hydroxyhexadecansäuremethylester besteht, als farbloses Oel, IR: 1719, 1361, 1254, 1076, 836, 774 $cm^{-1}$, aus einem Diastereomerengemisch, welches hauptsächlich aus (3S,5S)-5-(t-Butyldimethylsiloxy) -3-hydroxyhexadecansäuremethylester besteht.

2H. Herstellung von Estern der Formeln VIII und XV (in (S)-Form)

2H.a) 14,5 g eines Diastereomerengemisches, dessen Hauptkomponente (3S,5S)-5-(t-Butyldimethylsiloxy) -3-hydroxyhexadecansäure-(R)-2-hydroxy -1,2,2-triphenyläthylester ist, werden in 145 ml Methanol suspendiert. Man setzt 21,5 ml 1N methanolische Natriummethylatlösung zu und rührt 1 Stunde bei Raumtemperatur. Die Lösung wird auf 700 ml gesättigte Ammoniumchloridlösung gegossen. Es wird einmal mit 200 ml, dann zweimal mit 100 ml Diäthyläther extrahiert. Die vereinigten Extrakte werden getrocknet, filtriert und eingedampft. Der Rückstand wird in 100 ml n-Hexan aufgenommen und im Eisbad verrührt. Die wiessen Kristalle werden abgesaugt und das Filtrat wird eingedampft. Der Rückstand wird an Kieselgel chromatographiert. Man erhält ein Diastereomerengemisch als Oel, welches hauptsächlich aus (3S,5S)-5-(t-Butyldimethylsiloxy) -3-hydroxyhexadecansäure-methylester besteht, IR: 3464, 1739, 1255, 1171, 1087, 836, 775 $cm^{-1}$.

2H.b) Auf analoge Weise erhält man:

den (S)-3-Hydroxytetradecansäure-methylester, welcher etwa 15% des (R)-Enantiomeren enthält, Smp. 36-38°C,
aus (S)-3-Hydroxytetradecansäure-(R)-2-hydroxy -1,2,2-triphenyläthylester, welcher etwa 15% des (R)-Enantiomeren enthält.

2I. Herstellung von Estervorprodukten zu den Estern VIII und XV

2I.a) 9,75 g (R)-α-(Hydroxydiphenylmethyl)benzylacetat werden in 100 ml THF unter Argon auf -76°C abgekühlt. Dann wird eine Lösung von 2 Moläquivalenten Lithiumdiisopropylamid zugetropft. Man lässt auf 0°C aufwärmen und rührt 10 Minuten. Dann kühlt man erneut auf -76°C ab, tropft 10,05 g (S)-3-(t-Butyldimethylsiloxy)tetradecanal in 20 ml THF zu und rührt eine Stunde. Zur Hydrolyse versetzt man bei -76°C bis -70°C mit 25 ml gesättigter Ammoniumchloridlösung und lässt auf Raumtemperatur aufwärmen. Die wässrige Phase wird abgetrennt. Nach Waschen mit Wasser wird die organische Phase getrocknet, filtriert und eingedampft. Der Rückstand wird in Diäthyläther aufgenommen und verrührt. Unlösliches Material wird durch Filtration entfernt. Das Lösungsmittel wird im Vakuum entfernt und der Rückstand mehrmals aus Acetonitril umkristallisiert. Man erhält ein Diastereomerengemisch, dessen Hauptmenge aus (3S,5S)-(t-Butyldimethylsiloxy) -3-hydroxyhexadecansäure-(R)-2-hydroxy -1,2,2-triphenyläthylester besteht, Smp. 90-92°C.

2I.b) Auf analoge Weise erhält man (S)-3-Hydroxytetradecansäure-(R) -2-hydroxy-1,2,2-triphenyläthylester, welcher etwa 15% des 3-(R)-Isomeren enthält, vom Schmelzpunkt 112-115°C aus Dodecanal und (R)-α-(Hydroxydiphenylmethyl)benzylacetat.

2J. Herstellung eines Aldehyds der Formel VII (in (S)-Form)

In Analogie zu Beispiel 1J.a) erhält man (S)-3-(t.Butyldimethylsiloxy)tetradecanal, welcher etwa 15% des (R)-Enantiomeren enthält, als farbloses Oel vom Siedepunkt 132-140°/0,6 mm, aus(S)-3-(t-Butyldimethylsiloxy)tetradecansäure-methylester, welcher ebenfalls etwa 15% des (R)-Enantiomeren enthält.

2K. Herstellung eines Aethers der Formel XIII (in (S)-Form)

Man löst 12,9 g (S)-3-Hydroxytetradecansäuremethylester unter Argon in 50 ml DMF, setzt 9,0 g t-Butyldimethylchlorsilan zu, gibt portionenweise 8,5 g Imidazol zu und lässt 17 Stunden rühren. Das Reaktionsgemisch wird auf 300 ml Wasser gegossen und dreimal mit 50 ml Diäthyläther extrahiert. Die vereinigten organischen Phasen werden getrocknet, filtriert und eingedampft. Durch Destillation bei 140-145°C/0,07 mm erhält man (S)-3-(t-Butyldimethylsiloxy)tetradecansäuremethylester als farbloses Oel.

**Patentansprüche**

1. Verfahren zur Herstellung von Oxetanonen der Formel

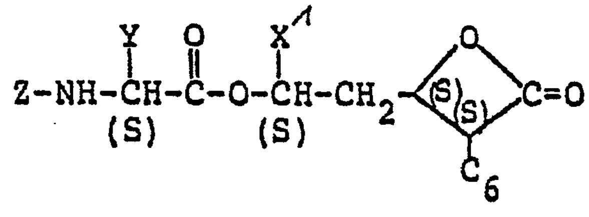

I

worin $X^1$ Undecyl oder 2Z,5Z-Undecadienyl, $C_6$ n-Hexyl, Y Isobutyl und Z Formyl, oder Y Carbamoylmethyl und Z Acetyl sind, dadurch gekennzeichnet, daß man

a) eine Säure der Formel

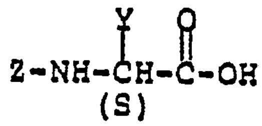

II

mit einem Alkohol der Formel

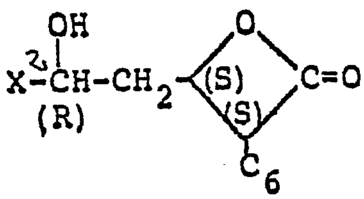

III

worin $X^2$ 3-Undecenyl oder wie $X^1$ ist, und $X^1$, $C_6$, Y und Z die obige Bedeutung haben, verestert, und ein gegebenenfalls erhaltenes Oxetanon der Formel

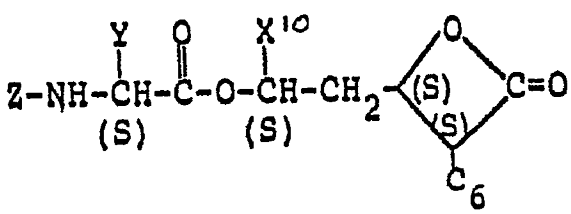

I'

worin $X^{10}$ 3-Undecenyl ist und $C_6$, Y und Z die obige Bedeutung haben,
hydriert, oder
b) ein Oxetanon der Formel

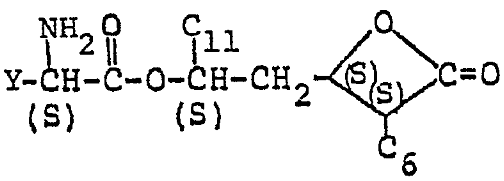

I''

worin $C_{11}$ Undecyl ist und Y und $C_6$ die obige Bedeutung haben,
mit einem die Gruppe Z einführenden Alkanoylierungsmittel behandelt.

2. Verfahren nach Anspruch 1, dadurch gekennzeichnet, daß man die Verfahrensvariante a) durchführt.

3. Verfahren nach Anspruch 1 oder 2 zur Herstellung des N-Formyl-L-leucin(S)-1-[[2S,3S)-3-hexyl-4-oxo-2-oxetanyl]methyl]dodecylesters.

**Claims**

1. A process for the manufacture of oxetanones of the formula

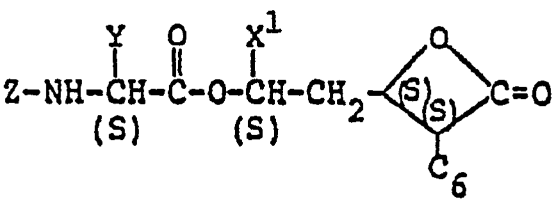

I

wherein $X^1$ is undecyl or 2Z,5Z-undecadienyl, $C_6$ is n-hexyl, Y is isobutyl and Z is formyl or Y is carbamoylmethyl and Z is acetyl, characterized by
a) esterifying an acid of the formula

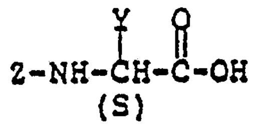

II

with an alcohol of the formula

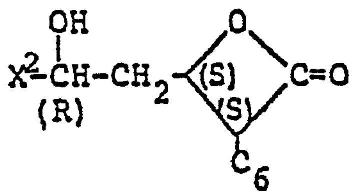

III

wherein $X^2$ is 3-undecenyl or is the same as $X^1$ and $X^1$, $C_6$, Y and Z have the above significance, and hydrogenating an oxetanone of the formula

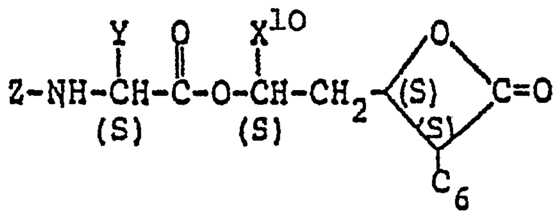

I'

wherein $X^{10}$ is 3-undecenyl and $C_6$, Y and Z have the above significance, which may be obtained, or

b) treating an oxetanone of the formula

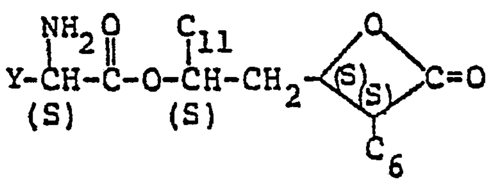

I"

wherein $C_{11}$ is undecyl and Y and $C_6$ have the above significance, with an alkanoylating agent which introduces the group Z.

2. A process according to claim 1, characterized in that process variant a) is carried out.

3. A process according to claim 1 or 2 for the manufacture of N-formyl-L-leucine (S)-1-[[(2S,3S)-3-hexyl-4-oxo-2-oxetanyl]methyl]dodecyl ester.

**Revendications**

1. Procédé de préparation d'oxétannones de formule:

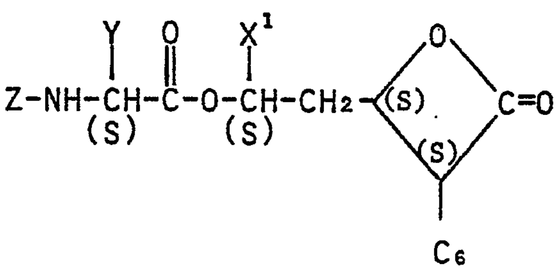

I

dans laquelle $X^1$ représente un groupe undécyle ou 2Z,5Z-undécadiényle, $C_6$ représente le groupe n-hexyle, Y représente un groupe isobutyle et Z un groupe formyle, ou bien Y représente un groupe carbamoylméthyle et Z un groupe acétyle, caractérisé en ce que:

a) on estérifie un acide de formule:

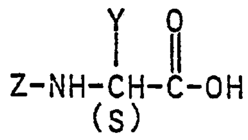

II

par un alcool de formule:

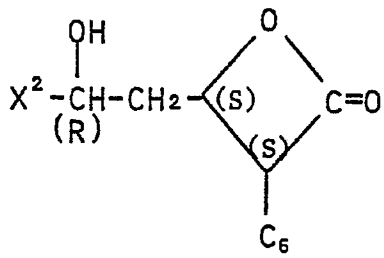

III

dans laquelle $X^2$ représente un groupe 3-undécényle ou a les mêmes significations que $X^1$, et $X^1$, $C_6$, Y et Z ont les significations indiquées ci-dessus, et on hydrogène une oxétannone éventuellement obtenue, répondant à la formule:

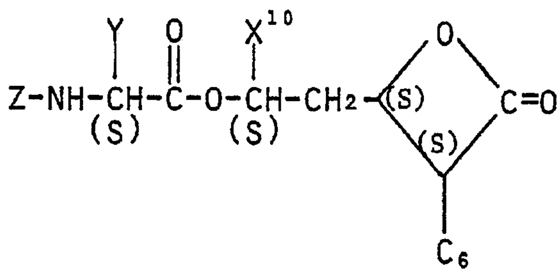

I'

dans laquelle $X^{10}$ représente un groupe 3-undécényle et $C_6$, Y et Z ont les significations indiquées ci-dessus, ou bien

b) on traite une oxétannone de formule:

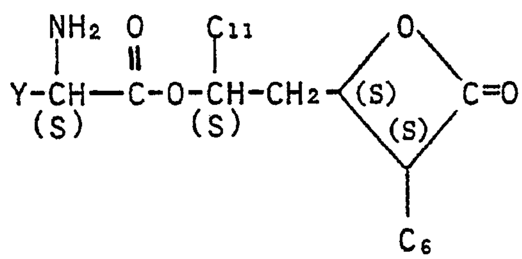

I"

dans laquelle $C_{11}$ représente un groupe undécyle et Y et $C_6$ ont les significations indiquées ci-dessus, par un agent alcanoylant introduisant le groupe Z.

2. Procédé selon la revendication 1, caractérisé en ce que l'on opère selon la variante a).

3. Procédé selon la revendication 1 ou 2, pour la préparation de l'ester (S)-1-[[2S,3S)-3-hexyl-4-oxo-2-oxétannyl]méthyl]-dodécylique de la N-formyl-L-leucine.